# EUROPÄISCHE PATENTSCHRIFT

(11) **EP 3 749 272 B1**
(45) Veröffentlichungstag und Bekanntmachung des Hinweises auf die Patenterteilung: **01.06.2022**
(21) Anmeldenummer: 19710641.2
(22) Anmeldetag: 07.03.2019
(51) Int. Cl.: A61G 1/00, A61G 1/04, A61F 7/08

(54) **TEMPERATURREGULIERENDE TRAGENAUFLAGE**
TEMPERATURE-REGULATING STRETCHER SUPPORT
BRANCARD THERMORÉGULATEUR

(30) Priorität: 09.03.2018 DE 202018101328 U
(43) Veröffentlichungstag der Anmeldung: 16.12.2020
(73) Patentinhaber: Körner, Andreas, 04769 Naundorf (DE)
(72) Erfinder: Körner, Andreas, 04769 Naundorf (DE)
(74) Vertreter: Manitz Finsterwald Patent- und Rechtsanwaltspartnerschaft mbB
(86) Internationale Anmeldenummer: PCT/EP2019/055740
(87) Internationale Veröffentlichungsnummer: WO 2019/170816

(56) Entgegenhaltungen:
- WO-A2-2013/086540
- DE-A1-102015 007 526
- DE-U1- 8 703 704

## Beschreibung

Die vorliegende Erfindung betrifft eine temperaturregulierende Tragenauflage, mit einem Kopfteil und einem Körperteil, die miteinander gekoppelt sind, mindestens einer temperaturregulierende Schicht und einer Stützlage.

Bei medizinischen Notfällen ist es generell notwendig einen Patienten schnell und effizient zu behandeln. Um Patienten in ein Krankenhaus zu transportieren, werden verschiedene Arten von Fahrzeugen verwendet. Hierbei kommen Krankentragen in den Fahrzeugen zum Einsatz.

Die derzeit gängigsten Tragenauflagen eignen sich in der Regel nur für die Verwendung innerhalb von Fahrzeugen oder Gebäuden und meist nicht für eine längere Verwendung außerhalb von Fahrzeugen und Gebäuden.

Um Tragenauflagen für den mobilen Einsatz außerhalb von Fahrzeugen bereitzustellen, werden temperaturregulierende Tragenauflagen verwendet. In Notfall-Situationen hat es sich gezeigt, dass durch das Zuführen von Kälte bzw. Wärme Patienten geholfen werden kann, da durch die Zufuhr von Wärme sich z. B. die Blutgefäße aufweiten können. Dadurch wird die Durchblutung angeregt und die Muskeln können entspannen. Auch kann durch die Zufuhr von Wärme Patienten nach einem medizinischen Notfall, bspw. einem Unfall oder einer akuten Erkrankung, durch die Zufuhr von Wärme geholfen werden. Eine temperaturregulierende Tragenauflage ist bekannt aus DE102015007526.

Im Gegensatz hierzu ziehen sich die Blutgefäße bei Zufuhr von Kälte zusammen und die Muskeln können sich anspannen. Hierbei kann der Austritt von Blut z. B. in umliegendes Gewebe verhindert werden. Durch die Regelung der Temperatur auf verschiedene Temperaturen kann somit ein Patient auch bei schwersten Verletzungen gezielter außerhalb von medizinischen Einrichtungen bzw. in oder außerhalb von Fahrzeugen behandelt werden.

Ein Nachteil solcher temperaturregulierenden Tragenauflagen ist, dass diese oftmals innerhalb des Fahrzeugs verkabelt werden und somit eine Gefahrenstelle im Fahrzeug bilden können. Auch durch die Verkabelung solcher temperaturregulierenden Tragenauflagen kann die Lebensdauer solcher Tragenauflagen beeinträchtigt werden.

Es ist eine Aufgabe der vorliegenden Erfindung, eine verbesserte temperaturregulierende Tragenauflage zu schaffen, die langlebiger und ohne Verkabelungsaufwand einsetzbar ist. Es ist eine weitere Aufgabe der vorliegenden Erfindung, eine Tragenauflage zu schaffen, die es ermöglicht einen Platzbedarf innerhalb eines Fahrzeugs zu minimieren.

Diese Aufgabe wird durch eine temperaturregulierende Auflage mit den Merkmalen des Anspruchs 1 gelöst.

Solch eine temperaturregulierende Tragenauflage wie bspw. eine medizinische Tragenauflage umfasst ein Kopfteil und ein Körperteil, die miteinander gekoppelt sind, mindestens eine temperaturregulierende Schicht und eine Stützlage, wobei die mindestens eine temperaturregulierende Schicht eine Batterie als Stromversorgung aufweist. Die temperaturregulierende Tragenauflage zeichnet sich dadurch aus, dass die Batterie in einem Fach der Tragenauflage angeordnet ist, und dass die Batterie, vorzugsweise wiederholbar, aus diesem Fach herausnehmbar und in dieses Fach einsetzbar ist.

Durch das Anordnen der Batterie in einem Fach der Tragenauflage kann sowohl die Batterie als auch die elektrische Verbindung zwischen der Batterie und der temperaturregulierende Schicht vor äußeren Einflüssen wie z.B. Flüssigkeiten, oder stolpern von medizinischem Personal besser geschützt werden. Durch das Ein- und Aussetzen der Batterie kann diese kabellos bspw. an einer Dockingstation geladen werden, was das Laden der Batterie vereinfacht.

Die Auflage wird generell im medizinischen Bereich beim Transport von Patienten verwendet und kann bevorzugt als Tragenauflage realisiert werden. Auch besteht die Möglichkeit die Auflage in einer Vakuummatratze oder in einem Transport.- bzw. Krankentransportstuhl zu integrieren.

Vorzugsweise ist das Fach und die Batterie im Kopfteil der Tragenauflage angeordnet.

Das Kopfteil einer Tragenauflage ist in der Regel starrer ausgebildet als der Körperteil und bietet somit einen verbesserten Schutz der Batterie gegenüber Stößen und Ähnlichem, die im Gebrauch der Tragenauflage auftretten können.

Besonders bevorzugt weist das Fach eine Abdeckung auf, womit die Batterie zusätzlich vor äußeren Einflüssen geschützt werden kann.

Bevorzugt ist die Abdeckung mit einer Oberseite der Tragenauflage fest verbunden und mit der Unterseite der Tragenauflage lösbar verbunden ist. Beim Reinigen der Tragenauflage wird typischerweise ein Desinfektionsmittel großflächig auf der Oberseite der Tragenauflage aufgetragen, durch die feste Verbindung der Abdeckung an der Oberseite kann ein Eindringen des Desinfektionsmittels ins Fach von oben besser verhindert werden. Durch die lösbare Verbindung an der Unterseite der Tragenauflage kann das Fach und somit die Batterie auf einfache Weise von außen erreicht werden.

Insbesondere umfasst die Tragenauflage zumindest eine magnetische Einrichtung, insbesondere einen Magneten, an der Unterseite der Tragenauflage, die mit einem magnetischen Gegenstück an der Abdeckung, kooperiert.

Vorzugsweise ist das Fach an die äußere Form der Batterie angepasst und die Tragenauflage, vorzugsweise das Fach, weist Mittel, bspw. mittels einer Feder vorgespannte Rastnasen oder Ähnliches, zum lösbaren Halten der Batterie auf. Somit kann die Batterie auf einfache Weise innerhalb der Tragenauflage gelagert werden.

Die temperaturregulierende Schicht ist auf eine zumindest im Wesentlichen konstante Temperatur im Bereich von 15 bis 42°C, bevorzugt von 16 bis 30°C, insbesondere von 20 bis 30°C, im Speziellen von 22 bis 26°C regelbar, wobei die Temperatur mittels einer Vorrichtung patientenabhängig eingestellt werden kann. Diese Temperaturbereiche werden von Patienten, die auf der Tragenauflage transportiert werden, als angenehm empfunden und ermöglichen ein Erwärmen bzw. ein Abkühlen des Körpers des Patienten.

Bevorzugt ist die Vorrichtung an der Tragenauflage angeordnet. Somit ist die Vorrichtung in direkter Nachbarschaft zu ihrem Einsatzort vorgesehen. In diesem Zusammenhang ist es insbesondere möglich die Vorrichtung am Kopfteil der Tragenauflage anzuordnen, da dieser Bereich der Tragenauflage bei einem sich bewegenden Fahrzeug am einfachsten zugänglich ist und durch die Anbringung am starreren Kopfteil auch besser geschützt ist als am Körperteil der Tragenauflage.

Insbesondere ist die Vorrichtung an einer Lasche angeordnet, die mit der Tragenauflage, insbesondere dem Kopfteil verbunden ist. Durch eine solche Lasche kann das Vorhandensein der temperaturregulierenden Tragenauflage besser sichtbar gemacht werden.

Erfindungsgemäß ist die Vorrichtung an der Batterie angeordnet, um Bauraum und ähnliches einzusparen.

Besonders bevorzugt umfasst die Vorrichtung eine Ladezustandsanzeige, einen An/Aus-Knopf, ein Bedienfeld zum Regeln der Temperatur und/oder einen Bildschirm. Solche Bedienelemente bzw. Anzeigeelemente vereinfachen das Verwenden der Tragenauflage. Somit kann bspw. der temperaturregulierende Betrieb der beheizbaren Tragenauflage auf einfache Weise an bzw. abgeschaltet werden, um z. B. bei Leerfahrten bzw. bei normalen Krankentransporten die Tragenauflage auszuschalten, wodurch Strom gespart werden kann. Somit können alle wesentlichen elektrischen Komponenten in bzw. an der Tragenauflage angeordnet werden und ein Ladezustand eines Akkus von außen sichtbar sein. Vorzugsweise ist die Vorrichtung mit der Batterie, der die temperaturregulierende Schicht sowie einer Regelschaltung verbunden. Hierbei wird die Stromversorgung aller elektrischen Bauteile der Tragenauflage mittels ein und derselben Batterie gewährleistet. Insbesondere kann die Regelschaltung einen Mikrokontroller umfassen, der es ermöglicht elektrische Signale, die an der Tragenauflage bereitgestellt werden, an weitere Geräte weiterzuleiten und/oder auszuwerten.

Vorzugsweise ist die Auflage und/oder die Batterie, insbesondere mittels der Regelschaltung, dazu ausgebildet ein akustisches und/oder visuelles Signal auszugeben, sobald eine vordefinierte Ladezustandsschwelle unterschritten ist, insbesondere wobei die vordefinierte Ladezustandsschwelle einen Ladeszustand von weniger als 20 %, vorzugsweise weniger als 10%, insbesondere weniger als 5 % der Batterie entspricht. Hierbei kann einem Benutzer der Auflage angezeigt werden, dass die Batterie aufgeladen werden muss.

Bevorzugt sind ein oder mehrere Sensoren in der Tragenauflage angeordnet, die dazu ausgebildet sind, patientenspezifische Parameter zu erfassen. Diese Sensoren können Zustandsparameter des sich auf der Tragenauflage befindlichen Patienten erfassen, ohne dass zusätzliche Sensoren von außen am Patienten angebracht werden müssen, was einen Verkabelungsaufwand innerhalb des Fahrzeugs deutlich minimiert und den Einsatzbereich der Tragenauflage gleichzeitig vergrößert.

Vorzugsweise sind ein oder mehrere medizinische Geräte in der Tragenauflage angeordnet. Die insbesondere dazu ausgebildet sind, in Abhängigkeit der patientenspezifischen Parameter, den Patienten zu behandeln, bspw. mittels eines Defribilators einen Patienten bei einem erkannten Herzstillstand zu reanimieren. Solch medizinische Geräte können vorzugsweise dazu ausgebildet sein, eine Behandlung am Patienten durchzuführen.

Besonders bevorzugt ist der Mikrokontroller dazu ausgebildet, die patientenspezifischen Parameter ggfs. nach einer Verarbeitung dieser, an eine Ausgabevorrichtung und/oder an das ein oder mehrere medizinische Geräte zu übermitteln, bspw. mittels Funk, Bluetooth, Wifi oder ähnlichen oder über eine sich an der Tragenauflage befindliche Schnittstelle auszugeben.

Vorzugsweise ist eine elektrische Verbindung zwischen der die temperaturregulierende Schicht, zumindest einem der ein oder mehreren Sensoren, dem einem oder mehreren medizinischem Gerät, der Batterie und der Regelschaltung innerhalb der Tragenauflage geführt. Durch das Führen der Verkabelung der elektrischen Bauteile der Tragenauflage innerhalb der Tragenauflage können diese besser vor äußeren Einflüssen geschützt werden.

Bevorzugt ist die elektrische Verbindung durch einen Übergang, der zwischen dem Kopfteil und dem Körperteil angeordnet ist, geführt. Somit kann sichergestellt werden, dass die Verkabelung der elektrischen Bauteile der Tragenauflage auch im Bereich des Übergangs vom Kopfteil zum Körperteil geschützt ist.

Besonders bevorzugt sind der ein oder mehrere Sensor und/oder das ein oder mehrere medizinische Gerät mit dem Mikrokontroller verbunden. Somit können die patientenspezifischen Parameter direkt an der Tragenauflage ausgewertet bzw. derart verarbeitet werden, dass ein auf der Tragenauflage befindlicher Patient behandelt werden kann.

Vorzugsweise sind der ein oder mehrere Sensor und/oder das ein oder mehrere medizinische Gerät im Körperteil angeordnet bzw. werden aus diesem herausgeführt, sodass diese mit dem Patienten verbunden werden können. Hierbei entsteht der kürzeste Messpfad für die verschiedenen messbaren patientenspezifischen Parameter. Auch müssen keine Kabel von bspw. einer Seitenwand des Fahrzeugs zum Patienten geführt werden, die bspw. in Notfallsituationen medizinisches Personal beeinträchtigen können.

Vorzugsweise ist der ein oder mehrere Sensor und/oder das ein oder mehrere medizinische Gerät dazu ausgebildet, eine Messung der patientenspezifischen Parameter und/oder eine Behandlung durch die Tragenauflage hindurch durchzuführen. hiermit kann ein Verkabelungsaufwand innerhalb eines Fahrzeugs noch weiter reduziert werden.

Es ist bevorzugt, wenn die Vorrichtung, insbesondere der Bildschirm der Vorrichtung, dazu ausgebildet ist, die patientenspezifischen Parameter anzuzeigen. Somit können die verschiedenen messbaren patientenspezifischen Parameter direkt an der Tragenauflage angezeigt werden.

Vorzugsweise ist die Ausgabevorrichtung eine separate Ausgabevorrichtung, an der die patientenspezifischen Parameter anzeigbar und/oder mittels derer die patientenspezifischen Parameter auswertbar sind. Bspw. kann das im Fahrzeug befindliche EKG dazu verwendet werden, die Parameter, die bspw. von EKG-Elektroden, die in der Tragenauflage integriert sind, gemessen werden, anzuzeigen. Die weiteren Parameter können an parameterspezifischen Einheiten angezeigt werden, und/oder aber auch an einer zentralen Einheit im Fahrzeug.

Besonders bevorzugt sind der ein oder mehrere Sensor dazu ausgebildet, Parameter zu erfassen, die ein EKG-Signal, einen Puls, einen Sauerstoffwert, eine Temperatur, einen MetHb-Wert, einen Zuckerwert und/oder einen CO-Wert eines Patienten umfassen, der auf der Tragenauflage angeordnet ist und/oder liegt. Generall kann der ein oder mehrer Sensor dazu ausgebildet sein, einen Vitalparameter eines Patienten, der mit der Auflage in Kontakt ist, zu erfassen und für eine weitere Verarbeitung zur Verfügung zu stellen.

Bevorzugterweise umfasst das ein oder mehrere medizinische Gerät einen Defibrillator, eine Sauerstoffversorgung, und/oder eine Saug- und/oder Spülvorrichtung. Diese sind Beispiele von medizinischen Geräten, die in Fahrzeugen häufig zum Einsatz gelangen.

Vorzugsweise ist die temperaturregulierende Tragenauflage mittels eines Smartdevice, wie bspw. eines Smartphone, eines Tablets, einer Smartwatch und/oder eine Smartbrille, ansteuerbar und/oder regelbar, wobei vorzugsweise die patientenspezifischen Parameter am Smartdevice anzeigbar und/oder auswertbar sind um ggf. eine Ansteuerung der temperaturregulierende Tragenauflage (10) durchzuführen. Somit kann bspw. ein Führer des Fahrzeugs die Temperatur der Tragenauflage in Abhängigkeit des Patienten anpassen und/oder seine Fahrweise auf dem Weg zu einer medizinischen Einrichtung in Abhängigkeit der gemessen Parameter anpassen und/oder eine medizinische Behandlung am Patienten in Abhängigkeit der gemessen Parameter anpassen.

Nach einer besonders bevorzugten Ausführungsform regelt die Vorrichtung die Temperatur der Tragenauflage auf eine zumindest im Wesentlichen konstante Temperatur für eine Dauer von 20 bis 60 Minuten. Die Dauer von 20 bis 60 Minuten ist ausreichend, um zumindest eine Erstvorsorgung im mobilen Einsatz durchführen zu können. Die Dauer des Einsatzes bei einer bestimmten Temperatur kann mittels der gewählten Batterie bestimmt werden.

Vorzugsweise umfasst das Fach ein Batteriegehäuse und die Batterie ist aus diesem Batteriegehäuse herausnehmbar und in dieses Batteriegehäuse wiederholbar einsetzbar. Mittels eines solchen Batteriegehäuses kann eine elektrische Verbindung zwischen der Batterie und den elektronischen Bauteile der Tragenauflage verbessert werden, auch kann ein Schutz vor eindringen einer Flüssigkeit in die Tragenauflage mittels eines solchen Batteriegehäuses verbessert werden.

Vorzugsweise umfasst das Batteriegehäuse ein mehrteiliges Gehäuse, welches zumindest Flüssigkeitsabweisend, bevorzugt Flüssigkeitsdicht ausgebildet ist. Dadurch dass das Batteriegehäuse flüssigkeitsabweisend ausgebildet werden kann, kann ein Eintritt von Flüssigkeit in das Batteriegehäuse vermieden werden, sodass die Gefahr eines Kurzschlusses in einem oder mehreren elektronischen Bauteilen, die in der Tragenauflage anordenbar sind, minimiert werden.

Vorzugsweise ist die Vorrichtung an einer äußeren sichtbaren Seite des Batteriegehäuses angeordnet. Somit kann die Vorrichtung an einem stabilen Rahmen angeordnet sein, sodass die Gefahr eines ungewollten Bruchs der Vorrichtung minimiert werden kann.

Vorzugsweise weist das Batteriegehäuse eine obere Schale, eine untere Schale sowie eine Blende auf, wobei die obere Schale mit der unteren Schale verbunden ist und die Blende mit der oberen Schale sowie mit der unteren Schale lösbar gekoppelt ist, insbesondere mittels einer lösbaren Schnappverbindung. Ein solches Batteriegehäuse kann einfach hergestellt werden, ermöglicht ein einfaches Einführen und Entfernen der Batterie und bietet zudem einen gewünschten Schutz vor äußeren Beeinträchtigungen, wie Flüssigkeit und Schlägen.

Vorzugsweise ist die Batterie mit der Blende verbunden und die Batterie ist zusammen mit der Blende aus diesem Batteriegehäuse herausnehmbar und in dieses Batteriegehäuse einsetzbar. Mittels einer solchen Blende kann die Batterie einfacher in dem Batteriegehäuse untergebracht werden.

Vorzugsweise weist die Blende einen Rahmen auf in der die Batterie angeordnet ist. Mittels eines solchen Rahmens kann die Batterie wiederholbar und präziser in das Batteriegehäuse eingeführt werden.

Vorzugsweise ist eine Dichtung zwischen der Blende und der oberen Schale sowie der unteren Schale angeordnet. Da die Blende in der Regel an der Außenseite der Auflage angeordnet ist, dichtet die Dichtung in der Regel ebenfalls derart von außen, dass keine Flüssigkeit, wie Wasser, Spülmittel, Desinfiziermittel, Blut oder ähnliches in die Tragenauflage gelangen kann.

Vorzugsweise ist/sind das Batteriegehäuse und/oder die Blende aus einem Plastik, vorzugsweise in einem Spritzgussverfahren, hergestellt. Hierdurch können die Bestandteile des Batteriegehäuses kostengünstig und reproduzierbar hergestellt werden.

Vorzugsweise ist die Dichtung zwischen der Blende und einer Nut eines äußeren Rahmens angeordnet, wobei der äußere Rahmen die obere Schale mit der unteren Schale verbindet. Durch einen solchen äußeren Rahmen kann die Verbindung zwischen den Gehäuseteilen des Batteriegehäuses verbessert werden.

Vorzugsweise ist die Vorrichtung im Bereich der Verbindung zwischen der oberen Schale sowie der unteren Schale angeordnet, vorzugsweise am äußeren Rahmen angeordnet. Hierdurch kann die Vorrichtung besonders sicher an dem Batteriegehäuse angebracht werden.

Vorzugsweise weist das Batteriegehäuse ferner einen Aufnahmeraum für zumindest eine Platine auf, auf der zumindest eine elektronische Schaltung vorgesehen ist, um mit der Batterie zu kommunizieren. Somit kann eine solche elektronische Schaltung sicherer in der Tragenauflage angeordnet werden, als ohne ein solches Batteriegehäuse.

Gemäß einem weiteren Aspekt betrifft die vorliegende Erfindung eine medizinische Tragenauflage umfassend ein oder mehrere Sensoren, und/oder ein oder mehrere medizinische Geräte, und einem Mikrokontroller, der dazu ausgebildet ist patientenspezifische Parameter zu verarbeiten, die anhand des ein oder mehreren Sensors messbar und/oder anhand des einem oder mehreren medizinischen Gerätes verarbeitbar sind.

Die Vorteile die vorstehend im Zusammenhang mit der temperaturregulierenden Tragenauflage diskutiert wurden gelten Sinngemäß für die medizinische Tragenauflage.

Weitere Ausführungsformen der Erfindung sind in der folgenden Figurenbeschreibung sowie in den Unteransprüchen angegeben.

Im Folgenden wird die Erfindung anhand von Ausführungsformen unter Bezugnahme auf die Figur im Detail beschrieben. Die Zeichnungen der Figur zeigen:
Fig. 1 einen schematischen Querschnitt durch eine erfindungsgemäße Tragenauflage,
Fig. 2 eine Ansicht von oben auf die Tragenauflage gemäß Fig. 1,
Fig. 3 ein Bedienfeld einer beispielhaften Tragenauflage,
Fig. 4 ein Bedienfeld einer beispielhaften Tragenauflage,
Fig. 5 ein Bedienfeld einer weiteren Ausführungsform einer Tragenauflage,
Fig. 6 eine Ansicht auf die Batterie der Tragenauflage der Fig. 5,
Fig. 7 eine schematische Ansicht der Tragenauflage der Fig. 5,
Fig. 8 eine Seitenansicht der Tragenauflage gemäß Fig. 4,
Fig. 9 eine Ansicht eines Batteriegehäuses,
Fig. 10 eine Seitenansicht auf das Batteriegehäuse der Fig. 9,
Fig. 11 eine Ansicht einer Batterie, die in einem Rahmen des Batteriegehäuses der Fig. 9 angeordnet ist,
Fig. 12 eine Ansicht des Batteriegehäuses der Fig. 9, bei dem eine obere Schale entfernt worden ist,
Fig. 13 eine Ansicht auf das Batteriegehäuse der Fig. 12, bei dem eine Platine entfernt worden ist und die Batterie nich komplett im Batteriegehäuse angeordnet ist, und
Fig. 14 eine Explosionszeichnung der Komponenten der Fig. 11.

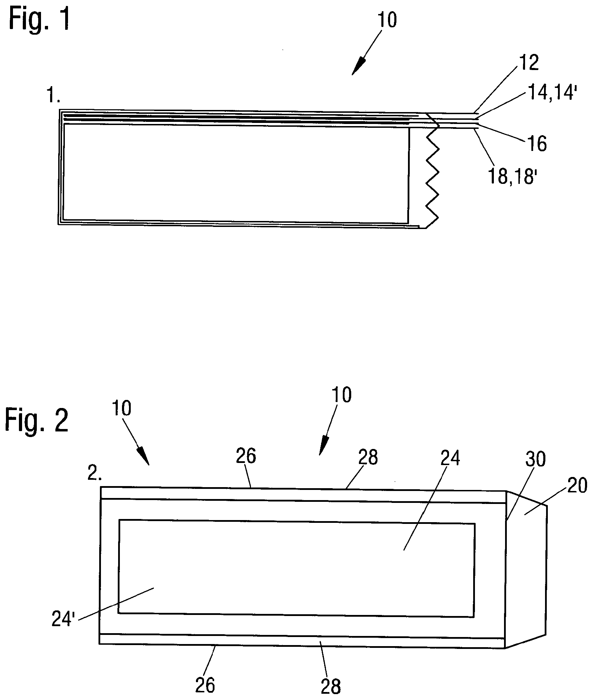

Die Fig. 1 zeigt einen schematischen Querschnitt durch eine erfindungsgemäße Tragenauflage 10. Die Tragenauflage 10 weist eine PVC Deckschicht 12 als Außenhaut auf, die eine Heizmatte 14 zumindest bereichsweise ummantelt. Unter der Heizmatte 14 befindet sich eine Stabilisierungsschicht 16, die auf einem Schaumkörper 18 angeordnet ist.

Die PVC Deckschicht 12 ist derart gewählt, dass diese keine Nässe durchlässt und auf einfache Weise, kostengünstig und schnell desinfizierbar ist. Die Stabilisierungsschicht 16 ist so gewählt, dass diese ein Verformen der Heizmatte 14 und somit ein Durchbrechen der darin befindlichen Heizelemente (nicht gezeigt) verhindert. Die Stabilisierungsschicht 16 kann z. B. aus Hartschaum oder Ähnlichem gefertigt werden. Ferner verhindert die Stabilisierungsschicht ein zu starkes Verformen der die temperaturregulierende Schicht, sodass diese langlebiger und bruchsicherer ausgebildet ist.

Der darunter liegende Schaumkörper 18 ist so gewählt, dass ein darauf transportierter Patient (nicht gezeigt) vor zu großen Erschütterungen geschützt ist und dadurch zumindest etwas Komfort genießen kann. Die Dichte des Schaumkörpers 18 kann individuell bestimmt werden, um die Tragenauflage 10 z. B. für die Verwendung in einem Hubschrauber (nicht gezeigt) vorzusehen. Der Schaumkörper 18 kann z. B. aus einem Kaltschaum gefertigt werden.

In diesem Zusammenhang soll es vermerkt sein, dass die oben beschriebenen Schichten 14, 16, und 18 auch in einer abweichenden Anordnung vorhanden sein können bzw. kann eine Schicht vorhanden sein, die die Funktionen der Schichten 16 und 18 in einer Lage übernimmt. Eine solche Schicht bildet dann die Stützlage 18' der Tragenauflage 10.

Die Tragenauflage 10 eignet sich insbesondere für Patienten mit einem Gewicht von bis zu ca. 250 kg, vorzugsweise jedoch bis zu 120 kg Körpergewicht.

Die Tragenauflage 10 kann ferner einen wasserundurchlässigen Reisverschluss umfassen (nicht gezeigt), der an einer Längsseite der Außenhaut oder an der Unterseite angeordnet sein kann, um die Möglichkeit zu schaffen, Bestandteile des Innenlebens, umfassend die Heizmatte, die Stabilisierungsschicht, die Schaumstofflage, elektrische Bauteile usw. auszutauschen bzw. zu warten.

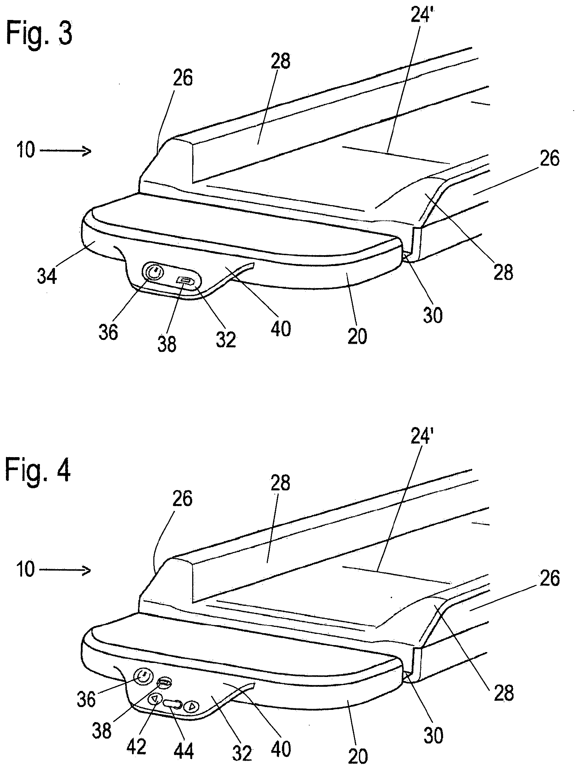

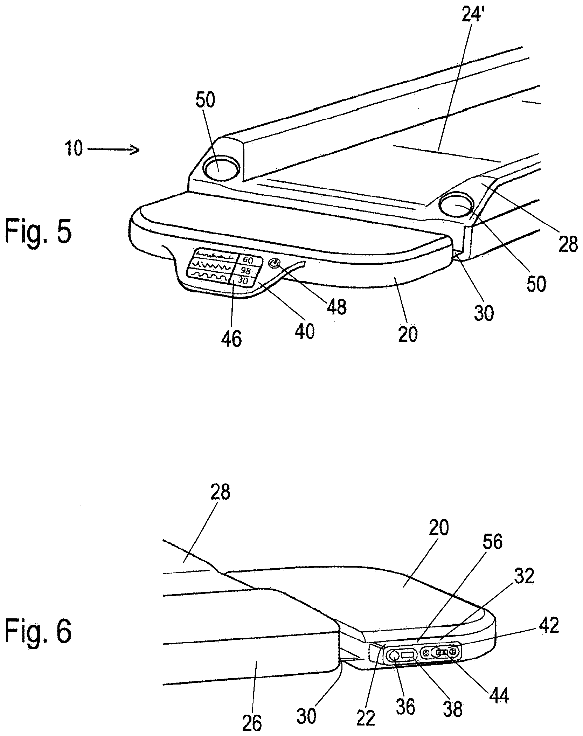

Die Heizmatte 14 bildet eine temperaturregulierende Schicht 14', die mittels einer Batterie 22 (siehe Fig. 6) mit Strom versorgt wird. Die temperaturregulierende Schicht 14' ist auf eine zumindest im Wesentlichen konstante Temperatur im Bereich von 16 bis 30°C, insbesondere von 20 bis 30°C, im Speziellen von 22 bis 26 bis 37°C regelbar, wobei die Temperatur mittels einer Vorrichtung 34' (siehe Fig. 3) patientenabhängig eingestellt werden kann.

Die Fig. 2 zeigt eine Ansicht von oben auf eine erfindungsgemäße Tragenauflage 10. In dem Kopfteil 20 ist die Batterie 22 (siehe Fig. 6) angeordnet. Die Heizmatte 14 ist in dem darunterliegenden Bereich 24, dem Körperteil 24' der Tragenauflage 10 angeordnet. Der Bereich 24 ist so gewählt, dass die die temperaturregulierende Schicht 14 auf die gewünschte Temperatur geregelt werden kann, sodass der Torso (nicht gezeigt) eines Patienten auf diesen temperierten Bereich zur temperaturunterstützten Behandlung gelegt werden kann.

An den Längsseiten 26 der Trageauflage 10 sind jeweils Stützbereiche 28 vorgesehen, um einen Patienten innerhalb dieser auf der Tragenauflage 10 gestützt zu halten. Die Abmessungen der Tragenauflage 10 sind jeweils für die geforderten Anforderungen bestimmbar.

Der temperaturgeregelte Bereich 24 erstreckt sich zumindest über 50% der Oberfläche der Tragenauflage 10. Vorzugsweise erstreckt sich der Temperatur geregelte Bereich 24 über 50 bis 90% der Oberfläche der Tragenauflage 10. Hierzu ist anzumerken, dass die Oberfläche der Tragenauflage 10 als der Bereich definiert ist, der durch Stützbereiche 28 und das Kopteil 20 begrenzt wird.

Der temperaturgeregelte Bereich 24 befindet sich in einem Körperteil 24', der mit dem Kopfteil gekoppelt ist. Diese Kopplung findet in einem Übergang 30 statt.

Fig. 3 zeigt ein Bedienfeld 34 der Vorrichtung 32 einer beispielhaften Tragenauflage 10. Die Vorrichtung 32 ist am Kopfteil 22 der Tragenauflage 10 angeordnet. Das Bedienfeld umfasst einen An/Aus-Knopf 36 sowie eine Ladezustandsanzeige 38. Die Vorrichtung 32 ist an einer Lasche 40 angeordnet, die mit dem Kopfteil 22 verbunden ist.

Die Fig. 4 zeigt ein weiteres Bedienfeld 34 einer weiteren beispielhaften Tragenauflage 10. Das Bedienfeld 34 umfasst zusätzlich zum An/Aus-Knopf 36 und der Ladezustandsanzeige 38 ein Bedienfeld 42 umfassend eine Temperaturanzeige 44 zum Regeln der Temperatur der Tragenauflage 10.

Fig. 5 zeigt einen Bildschirm 46 der an der Lasche 40 angeordnet ist. Im gezeigten Beispiel bildet der Bildschirm 46 ein EKG Signal ab, welches von einem Patienten (nicht gezeigt) gemessen werden kann. Ferner ist ein Drehknauf 48 sichtbar mittels dem zwischen verschiedenen Anzeigen des Bildschirms 46 gewechselt werden kann, um verschiedene patientenspezifische Parameter am Bildschirm 46 anzuzeigen.

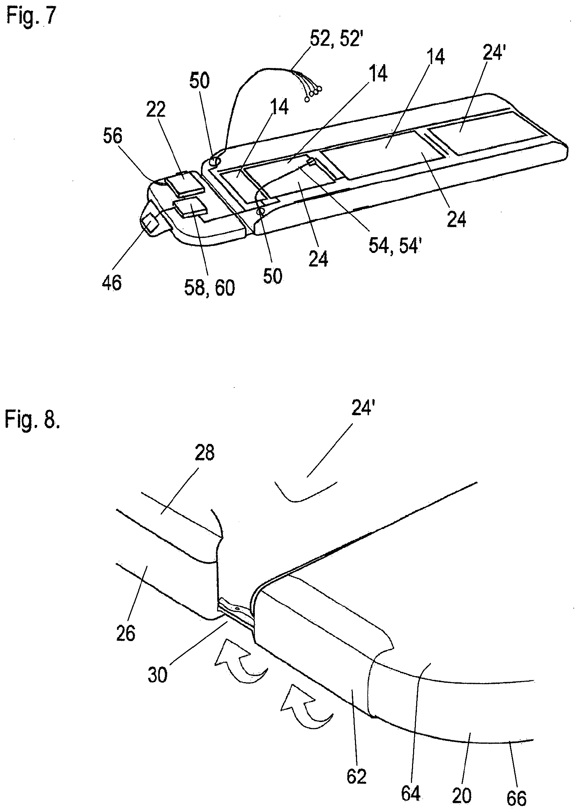

Die Seitenteile 28 der Fig. 5 zeigen jeweils eine Öffnung 50 einer Kabeldurchführung, durch die bspw. EKG-Kabel aus der linken Kabeldurchführung in der Fig. 5 herausgeführt werden können, um an einen Patienten angeschlossen zu werden. Aus der rechten Kabeldurchführung in der Fig. 5 kann bspw. ein Sauerstoffsensor herausgeführt werden, um mit dem Patienten verbunden zu werden. Wie im folgenden Beispiel der Fig. 7 beschrieben wird, kann die Tragenauflage 10 Sensoren 52 und 54 aufweisen.

Die Fig. 6 zeigt eine Ansicht auf die Batterie 22 der Tragenauflage 10 der Fig. 5. Die Vorrichtung 32 ist an der Batterie angeordnet und umfasst eine Ladezustandsanzeige 38, einen An/Aus-Knopf 36, sowie ein Bedienfeld 42 zum Regeln der Temperatur, dessen Einstellung an der Temperaturanzeige 44 abgelesen werden kann.

Die Batterie 22 ist herausnehmbar und wieder einsetzbar in einem Fach 56 der Tragenauflage 10 angeordnet. Das Fach 56 und die Batterie 22 befinden sich im Kopfteil 20 der Tragenauflage 10. Das Fach 56 ist an die äußere Form der Batterie 22 angepasst und die Tragenauflage 10 weist Mittel (nicht gezeigt) zum lösbaren Halten der Batterie 22 im Fach 56 auf.

Die Fig. 7 zeigt eine schematische Ansicht der Tragenauflage 10 der Fig. 5. Im vorliegenden Beispiel sind drei Heizmatten 14 im Körperteil 24' angeordnet, die zusammen den Temperatur geregelten Bereich 24 der Tragenauflage 10 bilden. Die Heizmatten 14 sind jeweils mit einer Regelschaltung 56, umfassend einen Mikrokontroller 58 verbunden. Die Regelschaltung ist ferner mit der Batterie 22 als Stromversorgung verbunden. Mittels zweier Sensoren 52, 54 werden patentspezifische Parameter gemessen, die mittels des Mikrokontrollers 58 verarbeitet bzw. ausgewertet werden können und von diesem an den Bildschirm 46 übermittelt werden und dort angezeigt werden können.

Eine elektrische Verbindung zwischen der die temperaturregulierende Schicht 14', der Sensoren 52, 54, der Batterie 22, des Bildschirms 46 und der Regelschaltung 58 ist innerhalb der Tragenauflage 10 geführt. Da die Batterie 22 und die Regelschaltung 58 im Kopfteil 20 angeordnet sind, ist die elektrische Verbindung zwischen der Regelschaltung 58 und der temperaturregulierende Schicht 14', bzw. vom Kopfteil 20 zum Körperteil 24' durch den Übergang 30, der zwischen dem Kopfteil 20 und dem Körperteil 24' angeordnet ist, geführt.

Weitere oder andere Sensoren als die Sensoren 52, 54 können in der Tragenauflage 10 integriert sein. Beispielsweise können solche Sensoren dazu ausgebildet sein, Parameter zu erfassen, die einen Puls, eine Temperatur, einen MetHb-Wert und/oder einen CO-Wert eines Patienten umfassen, der auf der Tragenauflage angeordnet ist und/oder liegt.

Zusätzlich zu oder als Alternative zu dem ein oder mehrerem Sensor könnten auch ein oder mehrere medizinische Geräte 52', 54' in der Tragenauflage 10 angeordnet sein. Diese können dazu ausgebildet sein, in Abhängigkeit der patientenspezifischen Parameter, den Patienten zu behandeln, bspw. mittels eines Defribilators einen Patienten bei einem Herzstillstand zu reanimieren. Das ein oder mehrere medizinische Gerät 52', 54' könnte bspw. einen Defibrillator, eine Sauerstoffversorgung, und/oder eine Saug- und/oder Spülvorrichtung oder ähnliches umfassen.

Die patientenspezifischen Parameter können mittels des Mikrokontrollers 60 an eine separate Ausgabevorrichtung (nicht gezeigt) übermittelt werden, wo diese anzeigbar und/oder auswertbar sind.

Die patientenspezifischen Parameter können mittels des Mikrokontrollers 60 auch an das ein oder mehrere medizinische Gerät übermittelt werden, um in Abhänigkeit der gemessenen patientenspezifischen Parameter eine Behandlung am patienten mittels des medizinische Geräts 52', 54' durchzuführen.

Bspw. ist die temperaturregulierende Tragenauflage 10 mittels eines Smartdevice (nicht gezeigt), wie bspw. eines Smartphone, eines Tablets, einer Smartwatch und/oder eine Smartbrille, als separate Ausgabevorrichtung zusätzlich ansteuerbar und/oder regelbar, wobei vorzugsweise die patientenspezifischen Parameter am Smartdevice anzeigbar und/oder auswertbar sind. Für diese Funktionen kann das Smartdevice eine eigens programmierte App aufweisen. Auch kann bspw. eine Behandlung, die bspw. mittels des medizinischen Geräts 52', 54' durchgeführt wird, durch ein solches Smartdevice angesteuert werden.

Die Fig. 8 zeigt eine Seitenansicht der Tragenauflage 10 gemäß Fig. 4. Das Fach 56 weist eine Abdeckung 62 auf, die mit einer Oberseite 64 der Tragenauflage 10 fest verbunden und mit der Unterseite 66 der Tragenauflage 10 lösbar verbunden ist.

Bspw. kann an der Abdeckung 62 ein Magnetband (nicht gezeigt) eingearbeitet sein, welches mit einem magnetischen Gegenstück an der Unterseite 66 der Tragenauflage 10 kooperiert, um die Abdeckung im Gebrauch zu halten. Die Abdeckung 62 wird im Gebrauch von der Untereseite 66 gelöst und relativ zur Oberseite verschenkt, um einen Zugang zum Fach 56 und der Batterie 22 zu ermöglichen. Im Gebrauch der Tragenauflage 10 entspricht die Oberseite 64 der Seite auf der der Patient angeordnet ist.

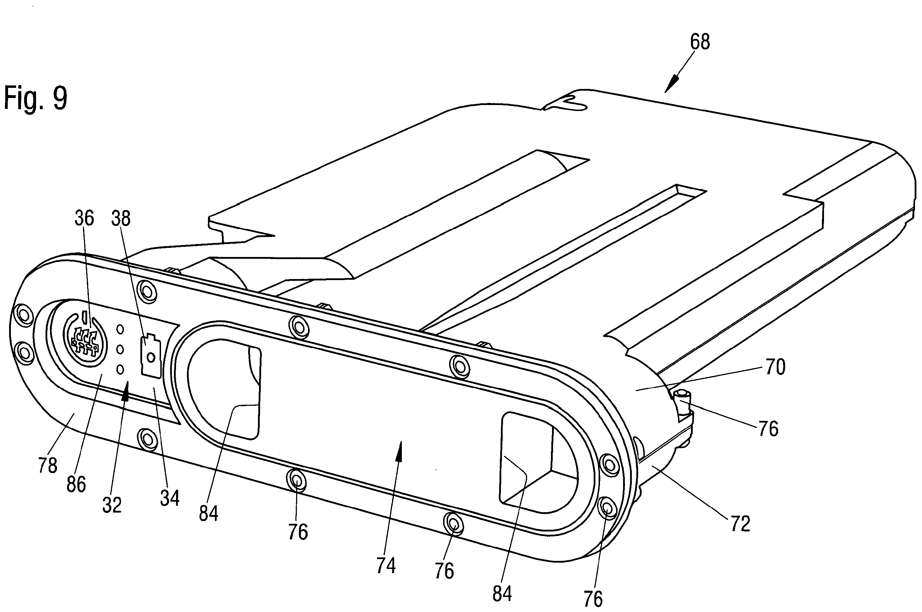

Fig. 9 zeigt eine Ansicht eines Batteriegehäuses 68. Das Batteriegehäuse 68 ist in dem Fach 58 fest eingebaut. Die Batterie 22 ist aus diesem Batteriegehäuse 68 sowohl herausnehmbar als auch wieder einsetzbar, bspw. um extern und separat von der Tragenauflage 10 aufgeladen bzw. gewartet zu werden.

Das Batteriegehäuse 68 ist ein mehrteiliges Gehäuse, welches zumindest Flüssigkeitsabweisend, bevorzugt Flüssigkeitsdicht ausgebildet ist, um einen Eintritt von Flüssigkeit im Bereich der Batterie in die Tragenauflage 10 zu verhindern.

Das Batteriegehäuse 68 weist eine obere Schale 70, eine untere Schale 72 sowie eine Blende 74 auf, wobei die obere Schale 70 mit der unteren Schale 72 verbunden ist. Um die Verbindung zwischen der oberen Schale 70 und der unteren Schale 78 zu verbessern sind Schraubverbindungen 76 vorgesehen. Ferner ist ein äußerer Rahmen 78 mittels Schraubverbindungen 76 mit der oberen Schale 70 und der unteren Schale 78 verbunden. Die Schraubverbindung umfasst typischerweise eine Schraube und eine Mutter, kann aber auch durch ein integrales Innen- oder Außengewinde gebildet werden, die dementsprechend mit einer Schraube oder einer Mutter kooperieren.

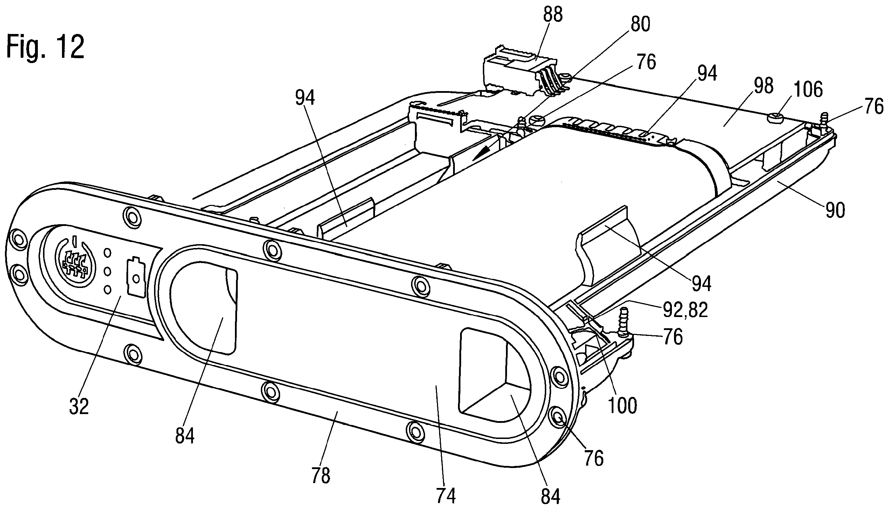

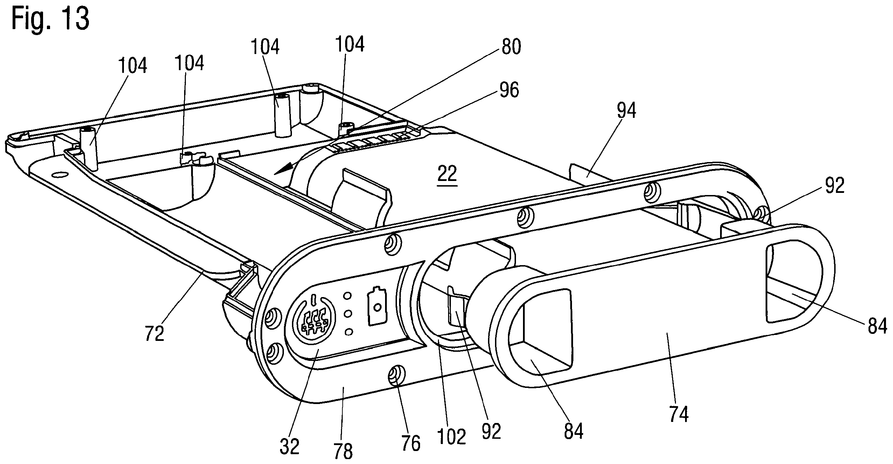

Die Blende 74 wird beim Zusammenbau durch den äußeren Rahmen 78 in einen Aufnahmeraum 80, der zwischen der oberen Schale 70 und der unteren Schale 72 ausgebildet ist, (siehe Fig. 12 und 13) eingeführt und mit der oberen Schale 70 sowie mit der unteren Schale 72 lösbar gekoppelt.

Die Kopplung zwischen dem Rahmen und der oberen Schale 70 sowie der unteren Schale 72 wird mittels einer lösbaren Schnappverbindung 82 (siehe Fig. 12) ermöglicht. Die Schnappverbindung 82 kann durch ein drücken in den Aussparungen 84 aktiviert werden, um die Verbindung bspw. zu lösen.

Die Vorrichtung 32 ist an einer äußeren sichtbaren Seite des Batteriegehäuses 68 angeordnet. Dies geschieht in dem eine Filmschaltung 86 auf dem das Bedienfeld 34, umfassend den An/Aus Knopf 36 sowie der Ladezustandsanzeige 38, angeordnet ist, mit der oberen Schale 70, der unteren Schale 72 und ggf. am Rahmen 78 befestigt ist.

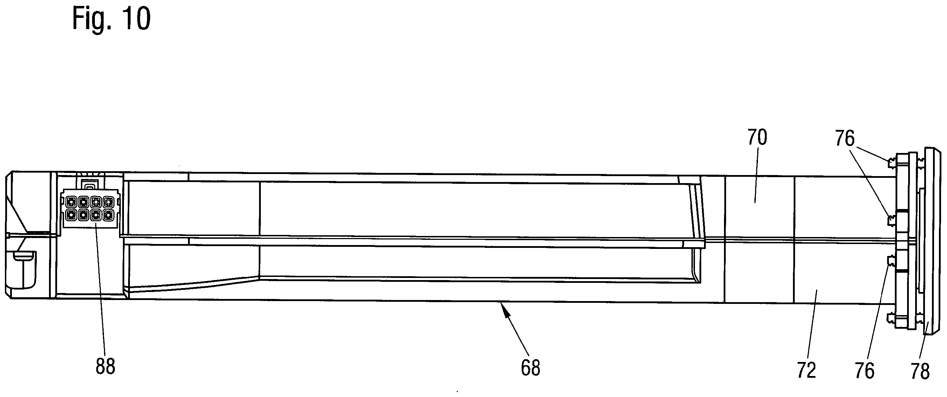

Die Fig. 10 zeigt eine Seitenansicht des Batteriegehäuses 68. An dem hinteren Ende des Batteriegehäuses 68, welches dem Rahmen 78 gegenüberliegt, ist eine elektrische Schnittstelle 88 vorgesehen. Mittels dieser Schnittstelle 88 können die elektronischen Bauteile der Tragenauflage 10, wie die Heizmatte 14, die Regelschaltung 58, der Mikrokontroller 60, die Sensoren 52, 54, und die medizinischen Geräte 52', 54' mit Strom versorgt werden.

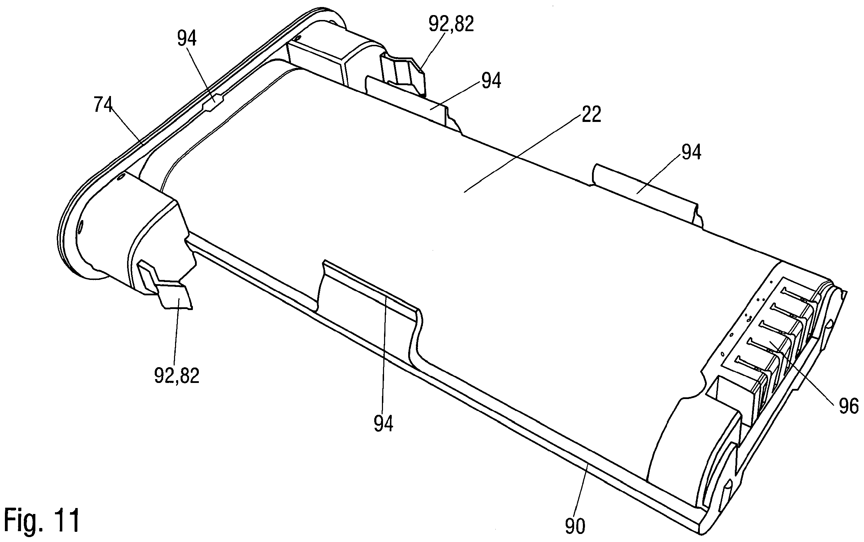

Die Fig. 11 zeigt eine Ansicht der Batterie 22, die in einem Rahmen 90 des Batteriegehäuses 68 angeordnet ist. Der Rahmen 90 ist hierzu an der Blende 74 befestigt. Genauer gesagt wird der Rahmen 90 zusammen mit der Blende 74 in einem Spritzgussverfahren aus Plastik hergestellt.

Um die Blende mit der oberen Schale 70 sowie der unteren Schale 72 zu verbinden sind Schnapphaken 92 an einem Teil der Blende 74 vorgesehen, auf dessen gegenüberliegenden Seite die Aussparungen 84 vorgesehen sind.

Die Batterie 22 ist mittels Rastnasen 94 sowohl mit der Blende 74 als auch dem Rahmen 90 formschlüssig verbunden. Die Batterie 22 kann zusammen mit der Blende 74 und dem Rahmen 90 aus dem Batteriegehäuse 68 herausgenommen als auch wieder eingesetzt werden.

An seinem der Blende 74 gegenüberliegenden Ende, weist die Batterie 22 eine Batterieschnittstelle 96 auf, mittels welcher die Batterie 22 mit der Schnittstelle 88 elektronisch verbunden werden kann.

Die Fig. 12 zeigt eine Ansicht des Batteriegehäuses 68, bei dem die obere Schale 70 entfernt worden ist. Im hinteren Ende des Batteriegehäuses 68 ist eine Platine 98 angeordnet, die die Batterie 22 über die Batterieschnittstelle 96 mit der Schnittstelle 88 elektronisch verbindet. Die Platine wird bspw. mittels Schrauben 106 an der unteren Schale 72 befestigt.

Ferner sind am vorderen Ende des Batteriegehäuses 68 zwei Aussparungen 100 vorgesehen, die dazu ausgebildet sind, mit den Schnapphacken 92 zu wechselwirken. In Fig. 12 ist einer dieser Aussparungen 100 durch den äußeren Rahmen 78 abgedeckt, sodass diese nicht sichtbar ist.

Die Fig. 13 zeigt eine Ansicht auf das Batteriegehäuse 68, bei dem die Platine 98 und die Schnittstelle 88 entfernt worden sind und bei dem die Batterie 22 leicht aus dem Batteriegehäuse 68 herausgezogen worden ist. Stege 104 sind am hinteren Ende des Batteriegehäuses 68 sichtbar mittels derer die Platine 98 mittels der Schrauben 106 an der unteren Schale 72 befestigt werden kann.

Der äußere Rahmen 78 weist eine Nut 102 auf, an der die Blende 74 bei eingeschobener Batterie 22 anliegt.

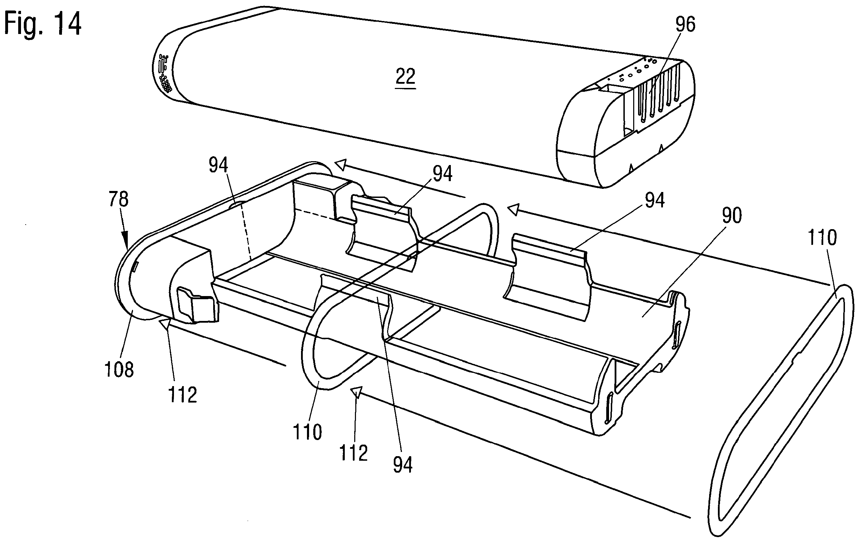

Fig. 14 zeigt eine Explosionszeichnung in der die Batterie 22 aus dem Rahmen 90 entfernt ist. Die Blende 74 weist eine Innenseite 108 auf, die eine ebene Fläche bildet. Ebenfalls ersichtlich ist eine Dichtung 110 die an dieser Innenseite 108 angeordnet wird, in dem diese in der Pfeilrichtung 112 auf dem Rahmen 90 aufgezogen wird. Diese Dichtung 110 ermöglicht eine Abdichtung zwischen der Blende 74, der oberen Schale 70 sowie der unteren Schale 72.

Die obere Schale 70, die untere Schale 72 als auch die Blende 74 werden aus einem Plastik, insbesondere einem Thermoplast, vorzugsweise in einem Spritzgussverfahren, hergestellt. Bspw. kann der Thermoplast aus der folgenden Liste von Thermoplasten ausgesucht werden: Acrylnitril-Butadien-Styrol (ABS), Polyamide (PA), Polylactat (PLA), Polymethylmethacrylat (PMMA), Polycarbonat (PC), Polyethylenterephthalat (PET), Polyethylen (PE), Polypropylen (PP), Polystyrol (PS), Polyetheretherketon (PEEK) und Polyvinylchlorid (PVC).

Das Batteriegehäuse 68 ist fest mit der Tragenauflage 10 verbunden und in dem Fach 56 angeordnet. Um eine herausfliegen der Batterie 22 aus dem Batteriegehäuse zu verhindem ist die Schnappverbindung 82 vorgesehen. Zusätzlich zu der Schnappverbindung 82 kann die Batterie 22 auch mittels der Abdeckung 62 in dem Fach 56 wie bspw. in der Fig. 8 gezeigt ist, gehalten werden.

### Bezugszeichenliste:

- 10: Tragenauflage
- 12: PVC Deckschicht
- 14: Heizmatte
- 16: Stabilisierungsschicht
- 18: Schaumkörper
- 20: Kopfteil
- 22: Batterie
- 24: Temperatur geregelter Bereich
- 26: Längseite
- 28: Stützbereich
- 30: Übergang
- 32: Vorrichtung
- 34: Bedienfeld
- 36: An/Aus-Knopf
- 38: Ladezustandsanzeige
- 40: Lasche
- 42: Bedienfeld
- 44: Temperaturanzeige
- 46: Bildschirm
- 48: Drehknauf
- 50: Öffnung der Kabeldurchführung
- 52, 52': Sensor, medizinisches Gerät
- 54, 54': Sensor, medizinisches Gerät
- 56: Fach
- 58: Regelschaltung
- 60: Mikrokontroller
- 62: Abdeckung
- 64: Oberseite
- 66: Unterseite
- 68: Batteriegehäuse
- 70: obere Schale
- 72: untere Schale
- 74: Blende
- 76: Schraubverbindung
- 78: Rahmen
- 80: Aufnahmeraum
- 82: Schnappverbindung
- 84: Aussparung
- 86: Filmschaltung
- 88: Schnittstelle
- 90: Rahmen
- 92: Schnapphacken
- 94: Rastnasen
- 96: Batterieschnittstelle
- 98: Platine
- 100: Aussparung
- 102: Nut
- 104: Steg
- 106: Schraube
- 108: Innenseite
- 110: Dichtung

## Patentansprüche

1. Temperaturregulierende Auflage, insbesondere Tragenauflage, (10), mit einem Kopfteil (20) und einem Körperteil (24'), die miteinander gekoppelt sind, mindestens einer temperaturregulierende Schicht (14'), und einer Stützlage (18'), wobei die mindestens eine temperaturregulierende Schicht (14') eine Batterie (22) als Stromversorgung aufweist, wobei die Batterie (22) in einem Fach (56) der Auflage (10) angeordnet ist, und die Batterie (22) aus diesem Fach (56) herausnehmbar und in dieses Fach (56) einsetzbar ist, wobei die Temperatur der temperaturregulierende Schicht (14') mittels einer Vorrichtung (32) patientenabhängig eingestellt werden kann, **dadurch gekennzeichnet, dass** die temperaturregulierende Schicht (14') auf eine zumindest im Wesentlichen konstante Temperatur im Bereich von 15 bis 42°C regelbar ist, und die Vorrichtung (32) an der Batterie (22) angeordnet ist.

2. Temperaturregulierende Auflage (10) nach Anspruch 1,
**dadurch gekennzeichnet, dass**
das Fach (56) und die Batterie (22) im Kopfteil (20) der Tragenauflage (10) angeordnet sind; und/oder, dass
das Fach (56) eine Abdeckung (62) aufweist, insbesondere wobei die Abdeckung (62) mit einer Oberseite (64) der Tragenauflage (10) fest verbunden und mit der Unterseite (66) der Tragenauflage (10) lösbar verbunden ist.

3. Temperaturregulierende Auflage (10) nach zumindest einem der vorstehenden Ansprüche,
**dadurch gekennzeichnet, dass**
das Fach (56) an die äußere Form der Batterie (22) angepasst ist und die Tragenauflage (10), vorzugsweise das Fach (56), Mittel zum lösbaren Halten der Batterie (22) aufweist;

4. Temperaturregulierende Auflage (10) nach zumindest einem der vorstehenden Ansprüche,
**dadurch gekennzeichnet, dass**
die Vorrichtung (32) eine Ladezustandsanzeige (38), einen An/Aus-Knopf (36), ein Bedienfeld (42) zum Regeln der Temperatur, eine Temperaturanzeige (44) und/oder einen Bildschirm (46) umfasst; und/oder
dass die Vorrichtung (32) mit der Batterie (22), der die temperaturregulierende Schicht (14') sowie einer Regelschaltung (58) verbunden ist, insbesondere wobei die Regelschaltung (58) einen Mikrokontroller (60) umfasst; und/oder dass ein Bildschirm (46) vorgesehen ist, der dazu ausgebildet ist, die patientenspezifischen Parameter anzuzeigen.

5. Temperaturregulierende Auflage (10) nach zumindest einem der vorstehenden Ansprüche,
**dadurch gekennzeichnet, dass**
ein oder mehrere Sensoren (52, 54) in der Auflage (10) angeordnet sind, die dazu ausgebildet sind, patientenspezifische Parameter zu erfassen.

6. Temperaturregulierende Auflage (10) nach zumindest einem der vorstehenden Ansprüche,
**dadurch gekennzeichnet, dass**
ein oder mehrere medizinische Geräte (52', 54') in der Tragenauflage (10) angeordnet sind.

7. Temperaturregulierende Auflage (10) nach Anspruch 5 und Anspruch 6,
**dadurch gekennzeichnet, dass**
das ein oder mehrere medizinische Geräte (52', 54') dazu ausgebildet ist, in Abhängigkeit der patientenspezifischen Parameter, den Patienten zu behandeln, bspw. mittels eines Defribilators einen Patienten bei einem Herzstillstand zu reanimieren.

8. Temperaturregulierende Auflage (10) nach den Ansprüchen 4 bis 7,
**dadurch gekennzeichnet, dass**
der Mikrokontroller (60) dazu ausgebildet ist, die patientenspezifische Parameter ggfs. nach einer Verarbeitung dieser, an eine Ausgabevorrichtung und/oder an das ein oder mehrere medizinische Gerät (52', 54') zu übermitteln; und/oder dass eine elektrische Verbindung zwischen der die temperaturregulierende Schicht (14'), zumindest einem der ein oder mehreren Sensoren (52, 54), dem einem oder mehreren medizinischem Gerät (52', 54'), der Batterie (22) und/oder der Regelschaltung (58) innerhalb der Auflage geführt ist, insbesondere wobei die elektrische Verbindung durch einen Übergang (30), der zwischen dem Kopfteil (20) und dem Körperteil (24') angeordnet ist, geführt ist; und/oder dass der ein oder mehrere Sensor (52, 54) und/oder dem einem oder mehreren medizinischem Gerät (52', 54') mit dem Mikrokontroller (60) verbunden ist; und/oder
dass der ein oder mehrere Sensor (52, 56) und/oder das ein oder mehrere medizinische Gerät (52', 54') im Körperteil (24') angeordnet und/oder aus diesem herausführbar ist, vorzugsweise wobei der ein oder mehrere Sensor (52, 56) und/oder das ein oder mehrere medizinische Gerät (52', 54') dazu ausgebildet ist, eine Messung der patientenspezifischen Parameter und/oder eine Behandlung durch die Auflage hindurch durchzuführen; und/oder,
dass die Ausgabevorrichtung eine separate Ausgabevorrichtung ist, an der die patientenspezifischen Parameter anzeigbar und/oder mittels derer die patientenspezifischen Parameter auswertbar sind.

9. Temperaturregulierende Auflage (10) nach einem der Ansprüche 5 bis 8,
**dadurch gekennzeichnet, dass**
der ein oder mehrere Sensor (52, 54) dazu ausgebildet ist, Parameter zu erfassen, die ein EKG Signal, einen Puls, einen Sauerstoffwert, eine Temperatur, einen MetHb Wert, einen Zuckerwert und/oder einen CO Wert eines Patienten umfassen, der auf der Tragenauflage angeordnet ist und/oder liegt; und/oder,
dass das ein oder mehrere medizinische Gerät (52', 54') einen Defibrillator, eine Sauerstoffversorgung, und/oder eine Saug- und/oder Spülvorrichtung umfasst.

10. Temperaturregulierende Auflage (10) nach zumindest einem der vorstehenden Ansprüche,
**dadurch gekennzeichnet, dass**
die temperaturregulierende Auflage (10) mittels eines Smartdevice, wie bspw. eines Smartphone, eines Tablets, einer Smartwatch und/oder eine Smartbrille, ansteuerbar und/oder regelbar ist, wobei vorzugsweise die patientenspezifischen Parameter am Smartdevice anzeigbar und/oder auswertbar sind, um ggf. eine Ansteuerung der temperaturregulierende Auflage (10) durchzuführen.

11. Temperaturregulierende Auflage (10) nach zumindest einem der vorstehenden Ansprüche,
**dadurch gekennzeichnet, dass**
das Fach (56) ein Batteriegehäuse (68) umfasst und, dass die Batterie (22) aus diesem Batteriegehäuse (68) herausnehmbar und in dieses Batteriegehäuse (68) einsetzbar ist.

12. Temperaturregulierende Auflage (10) nach Anspruch 11,
**dadurch gekennzeichnet, dass**
das Batteriegehäuse (68) ein mehrteiliges Gehäuse (70, 72, 74, 78, 90) umfasst, welches zumindest Flüssigkeitsabweisend, bevorzugt Flüssigkeitsdicht ausgebildet ist; und/oder,
dass die Vorrichtung (32) an einer äußeren sichtbaren Seite des Batteriegehäuses (68) angeordnet ist; und/oder,
dass das Batteriegehäuse (68) und/oder die Blende (74) aus einem Plastik, vorzugsweise in einem Spritzgussverfahren, hergestellt ist/sind; und/oder,
dass das Batteriegehäuse (68) ferner einen Aufnahmeraum (80) für zumindest eine Platine (98) aufweist auf der zumindest eine elektronische Schaltung vorgesehen ist, um mit der Batterie (22) zu kommunizieren.

13. Temperaturregulierende Auflage (10) nach Anspruch 11 oder 12,
**dadurch gekennzeichnet, dass**
das Batteriegehäuse (68) eine obere Schale (70), eine untere Schale (72) sowie eine Blende (74) aufweist, wobei die obere Schale (70) mit der unteren Schale (72) verbunden ist und die Blende (74) mit der oberen Schale (70) sowie mit der unteren Schale (72) lösbar gekoppelt ist, insbesondere mittels einer lösbaren Schnappverbindung (82).

14. Temperaturregulierende Auflage (10) nach Anspruch 13,
**dadurch gekennzeichnet, dass**
die Batterie (22) mit der Blende (74) verbunden ist und dass die Batterie (22) zusammen mit der Blende (74) aus diesem Batteriegehäuse (68) herausnehmbar und in dieses Batteriegehäuse (68) einsetzbar ist; insbesondere wobei die Blende (74) einen Rahmen (90) aufweist in der die Batterie (22) angeordnet ist; und/oder, dass eine Dichtung (110) zwischen der Blende (74) und der oberen Schale (70) sowie der unteren Schale (72) angeordnet ist, insbesondere wobei die Dichtung (110) zwischen der Blende (74) und einer Nut (102) eines äußeren Rahmens (78) angeordnet ist, wobei der äußere Rahmen (78) die obere Schale (70) mit der unteren Schale (72) verbindet.

15. Temperaturregulierende Auflage (10) nach einem der Ansprüche 12 bis 14,
**dadurch gekennzeichnet, dass**
die Vorrichtung (32) im Bereich der Verbindung zwischen der oberen Schale (70) sowie der unteren Schale (72) angeordnet ist, vorzugsweise am äußeren Rahmen (78) angeordnet ist.

## Claims

1. A temperature-regulating support, in particular a stretcher support (10), comprising a head part (20) and a body part (24') which are coupled to one another; at least one temperature-regulating layer (14'); and a support layer (18'), wherein the at least one temperature-regulating layer (14') has a battery (22) as a power supply, wherein the battery (22) is arranged in a compartment (56) of the support (10) and the battery (22) can be removed from said compartment (56) and can be inserted into said compartment (56), wherein the temperature of the temperature-regulating layer (14') can be set in dependence on the patient by means of an apparatus (32), **characterized in that** the temperature-regulating layer (14') can be regulated to an at least substantially constant temperature in the range from 15 to 42°C and the apparatus (32) is arranged at the battery (22).

2. A temperature-regulating support (10) in accordance with claim 1,
**characterized in that**
the compartment (56) and the battery (22) are arranged in the head part (20) of the stretcher support (10); and/or **in that**
the compartment (56) has a covering (62), in particular with the covering (62) being fixedly connected to an upper side (64) of the stretcher support (10) and being releasably connected to the lower side (66) of the stretcher support (10).

3. A temperature-regulating support (10) in accordance with at least one of the preceding claims,
**characterized in that**
the compartment (56) is adapted to the outer shape of the battery (22) and the stretcher support (10), preferably the compartment (56), has means for releasably holding the battery (22).

4. A temperature-regulating support (10) in accordance with at least one of the preceding claims,
**characterized in that**
the apparatus (32) comprises a charge state display (38); an on/off button (36); a control panel (42) for regulating the temperature; a temperature display (44); and/or a screen (46); and/or
**in that** the apparatus (32) is connected to the battery (22), to the temperature-regulating layer (14') and to a regulation circuit (58), in particular with the regulation circuit (58) comprising a microcontroller (60); and/or
**in that** a screen (46) is provided which is configured to display the patient-specific parameters.

5. A temperature-regulating support (10) in accordance with at least one of the preceding claims,
**characterized in that**
one or more sensors (52, 54) are arranged in the support (10) and are configured to detect patient-specific parameters.

6. A temperature-regulating support (10) in accordance with at least one of the preceding claims,
**characterized in that**
one or more medical devices (52', 54') are arranged in the stretcher support (10).

7. A temperature-regulating support (10) in accordance with claim 5 and claim 6,
**characterized in that**
one or more medical devices (52', 54') are configured to treat the patient in dependence on the patient-specific parameters, for example, to resuscitate a patient by means of a defibrillator in the event of a cardiac arrest.

8. A temperature-regulating support (10) in accordance with the claims 4 to 7,
**characterized in that**
the microcontroller (60) is configured to transmit the patient-specific parameters, if necessary after processing them, to an output apparatus and/or to the one or more medical devices (52', 54'); and/or
**in that** an electrical connection between the temperature-regulating layer (14'), at least one of the one or more sensors (52, 54), the one or more medical devices (52', 54'), the battery (22) and/or the regulation circuit (58) is led within the support, in particular with the electrical connection being led through a transition (30) which is arranged between the head part (20) and the body part (24'); and/or
**in that** the one or more sensors (52, 54) and/or the one or more medical devices (52', 54') is/are connected to the microcontroller (60); and/or
**in that** the one or more sensors (52, 56) and/or the one or more medical devices (52', 54') is/are arranged in the body part (24') and/or can be led out of it, preferably with the one or more sensors (52, 56) and/or the one or more medical devices (52', 54') being configured to perform a measurement of the patient-specific parameters and/or a treatment through the support; and/or
**in that** the output apparatus is a separate output apparatus at which the patient-specific parameters can be displayed and/or by means of which the patient-specific parameters can be evaluated.

9. A temperature-regulating support (10) in accordance with any one of the claims 5 to 8,
**characterized in that**
the one or more sensors (52, 54) is/are configured to detect parameters which comprise an ECG signal; a pulse; an oxygen value; a temperature; a MetHb value; a sugar value; and/or a CO value of a patient who is placed and/or lies on the stretcher support; and/or
**in that** the one or more medical devices (52', 54') comprises/comprise a defibrillator; an oxygen supply; and/or a suction and/or flushing apparatus.

10. A temperature-regulating support (10) in accordance with at least one of the preceding claims,
**characterized in that**
the temperature-regulating support (10) can be controlled and/or regulated by means of a smart device such as, for example, a smart phone, a tablet, a smart watch and/or smart glasses, with preferably the patient-specific parameters being able to be displayed and/or evaluated at the smart device in order, if necessary, to perform a control of the temperature-regulating support (10).

11. A temperature-regulating support (10) in accordance with at least one of the preceding claims,
**characterized in that**
the compartment (56) comprises a battery housing (68); and **in that** the battery (22) can be removed from said battery housing (68) and can be inserted into said battery housing (68).

12. A temperature-regulating support (10) in accordance with claim 11,
**characterized in that**
the battery housing (68) comprises a multi-part housing (70, 72, 74, 78, 90) which is at least liquid-repellent, preferably liquid-tight; and/or
**in that** the apparatus (32) is arranged at an outer visible side of the battery housing (68); and/or
**in that** the battery housing (68) and/or the cover (74) is/are manufactured from a plastic, preferably in an injection molding process; and/or
**in that** the battery housing (68) further has a reception space (80) for at least one board (98) on which at least one electronic circuit is provided to communicate with the battery (22).

13. A temperature-regulating support (10) in accordance with claim 11 or claim 12,
**characterized in that**
the battery housing (68) has an upper shell (70), a lower shell (72) and a cover (74), with the upper shell (70) being connected to the lower shell (72) and the cover (74) being releasably coupled to the upper shell (70) and to the lower shell (72), in particular by means of a releasable snap-in connection (82).

14. A temperature-regulating support (10) in accordance with claim 13,
**characterized in that**
the battery (22) is connected to the cover (74); and **in that** the battery (22) can be removed from said battery housing (68) and can be inserted into said battery housing (68) together with the cover (74), in particular with the cover (74) having a frame (90) in which the battery (22) is arranged; and/or in that a seal (110) is arranged between the cover (74) and the upper shell (70) as well as the lower shell (72), in particular with the seal (110) being arranged between the cover (74) and a groove (102) of an outer frame (78), with the outer frame (78) connecting the upper shell (70) to the lower shell (72).

15. A temperature-regulating support (10) in accordance with any one of the claims 12 to 14,
**characterized in that**
the apparatus (32) is arranged in the region of the connection between the upper shell (70) and the lower shell (72), preferably arranged at the outer frame (78).

## Revendications

1. Support thermorégulateur, en particulier brancard (10), comportant une partie de tête (20) et une partie de corps (24') qui sont couplées l'une à l'autre, au moins une couche thermorégulatrice (14') et une couche d'appui (18'), ladite au moins une couche thermorégulatrice (14') comprenant une batterie (22) comme alimentation électrique, la batterie (22) étant disposée dans un compartiment (56) du support (10), et la batterie (22) pouvant être retirée de ce compartiment (56) et être insérée dans ce compartiment (56), la température de la couche thermorégulatrice (14') pouvant être réglée en fonction du patient au moyen d'un dispositif (32),
**caractérisé en ce que**
la couche thermorégulatrice (14') peut être régulée à une température au moins sensiblement constante dans la plage de 15 à 42°C, et le dispositif (32) est disposé sur la batterie (22).

2. Support thermorégulateur (10) selon la revendication 1,
**caractérisé en ce que**
le compartiment (56) et la batterie (22) sont disposés dans la partie de tête (20) du brancard (10) ; et/ou **en ce que**
le compartiment (56) comprend un couvercle (62), en particulier le couvercle (62) étant solidaire d'une face supérieure (64) du brancard (10) et étant relié de manière amovible à la face inférieure (66) du brancard (10).

3. Support thermorégulateur (10) selon l'une au moins des revendications précédentes,
**caractérisé en ce que**
le compartiment (56) est adapté à la forme extérieure de la batterie (22), et le brancard (10), de préférence le compartiment (56), comprend des moyens pour maintenir de manière amovible la batterie (22).

4. Support thermorégulateur (10) selon l'une au moins des revendications précédentes,
**caractérisé en ce que**
le dispositif (32) comprend un indicateur d'état de charge (38), un bouton marche/arrêt (36), un panneau de commande (42) pour réguler la température, un indicateur de température (44) et/ou un écran d'affichage (46) ; et/ou
le dispositif (32) est relié à la batterie (22), à la couche thermorégulatrice (14') ainsi qu'à un circuit de régulation (58), en particulier le circuit de régulation (58) comprenant un microcontrôleur (60) ; et/ou il est prévu un écran d'affichage (46) qui est réalisé pour afficher les paramètres spécifiques au patient.

5. Support thermorégulateur (10) selon l'une au moins des revendications précédentes,
**caractérisé en ce que**
un ou plusieurs capteurs (52, 54) sont disposés dans le support (10) et sont réalisés pour détecter des paramètres spécifiques au patient.

6. Support thermorégulateur (10) selon l'une au moins des revendications précédentes,
**caractérisé en ce que**
un ou plusieurs dispositifs médicaux (52', 54') sont disposés dans le brancard (10).

7. Support thermorégulateur (10) selon la revendication 5 et la revendication 6,
**caractérisé en ce que**
un ou plusieurs dispositifs médicaux (52', 54') sont réalisés pour traiter le patient en fonction des paramètres spécifiques au patient, par exemple pour réanimer un patient en arrêt cardiaque au moyen d'un défibrillateur.

8. Support thermorégulateur (10) selon les revendications 4 à 7,
**caractérisé en ce que**
le microcontrôleur (60) est réalisé pour transmettre les paramètres spécifiques au patient, le cas échéant après traitement de ceux-ci, à un dispositif de sortie et/ou audit ou auxdits plusieurs dispositifs médicaux (52', 54') ; et/ou
une connexion électrique entre la couche thermorégulatrice (14'), l'un au moins dudit ou desdits capteurs (52, 54), ledit ou lesdits dispositifs médicaux (52', 54'), la batterie (22) et/ou le circuit de régulation (58) est menée à l'intérieur du support, en particulier la connexion électrique étant menée à travers un passage (30) disposé entre la partie de tête (20) et la partie de corps (24') ; et/ou
ledit ou lesdits capteurs (52, 54) et/ou ledit ou lesdits dispositifs médicaux (52', 54') sont reliés au microcontrôleur (60) ;
et/ou
ledit ou lesdits capteurs (52, 56) et/ou ledit ou lesdits dispositifs médicaux (52', 54') sont disposés dans la partie de corps (24') et/ou peuvent en être extraits, de préférence ledit ou lesdits capteurs (52, 56) et/ou ledit ou lesdits dispositifs médicaux (52', 54') étant réalisés pour effectuer une mesure des paramètres spécifiques au patient et/ou un traitement à travers le support ; et/ou
le dispositif de sortie est un dispositif de sortie séparé sur lequel les paramètres spécifiques au patient peuvent être affichés et/ou au moyen duquel les paramètres spécifiques au patient peuvent être évalués.

9. Support thermorégulateur (10) selon l'une des revendications 5 à 8,
**caractérisé en ce que**
ledit ou lesdits capteurs (52, 54) sont réalisés pour détecter des paramètres incluant un signal ECG, un pouls, une valeur d'oxygène, une température, une valeur de MetHb, une valeur de sucre et/ou une valeur de CO d'un patient placé et/ou allongé sur le brancard ; et/ou
ledit ou lesdits dispositifs médicaux (52', 54') comprennent un défibrillateur, une alimentation en oxygène et/ou un dispositif d'aspiration et/ou de rinçage.

10. Support thermorégulateur (10) selon l'une au moins des revendications précédentes,
**caractérisé en ce que**
le support thermorégulateur (10) peut être piloté et/ou contrôlé au moyen d'un appareil intelligent, comme par exemple un Smartphone, une tablette, une montre intelligente et/ou des lunettes intelligentes, de préférence les paramètres spécifiques au patient pouvant être affichés et/ou évalués sur l'appareil intelligent afin d'effectuer le cas échéant un pilotage du support thermorégulateur (10).

11. Support thermorégulateur (10) selon l'une au moins des revendications précédentes,
**caractérisé en ce que**
le compartiment (56) comprend un boîtier de batterie (68), et
**en ce que** la batterie (22) peut être retirée de ce boîtier de batterie (68) et être insérée dans ce boîtier de batterie (68).

12. Support thermorégulateur (10) selon la revendication 11,
**caractérisé en ce que**
le boîtier de batterie (68) comprend un boîtier en plusieurs pièces (70, 72, 74, 78, 90) qui est réalisé au moins pour repousser les liquides, de préférence pour être étanche aux liquides ; et/ou
**en ce que** le dispositif (32) est disposé sur une face extérieure visible du boîtier de batterie (68) ; et/ou
**en ce que** le boîtier de batterie (68) et/ou le cache (74) est/sont fabriqué(s) en plastique, de préférence par un procédé de coulée par injection ; et/ou en ce que le boîtier de batterie (68) comprend en outre un espace de logement (80) pour au moins une platine (98) sur laquelle est prévu au moins un circuit électronique pour communiquer avec la batterie (22).

13. Support thermorégulateur (10) selon la revendication 11 ou 12,
**caractérisé en ce que**
le boîtier de batterie (68) comprend une coque supérieure (70), une coque inférieure (72) ainsi qu'un cache (74), la coque supérieure (70) étant reliée à la coque inférieure (72) et le cache (74) étant couplé de manière amovible à la coque supérieure (70) ainsi qu'à la coque inférieure (72), en particulier au moyen d'une liaison par encliquetage (82) amovible.

14. Support thermorégulateur (10) selon la revendication 13,
**caractérisé en ce que**
la batterie (22) est reliée au cache (74), et
**en ce que** la batterie (22), ensemble avec le cache (74), peut être retirée de ce boîtier de batterie (68) et être insérée dans ce boîtier de batterie (68) ; en particulier, le cache (74) comprenant un cadre (90) dans lequel est disposée la batterie (22) ; et/ou
**en ce qu'**un joint d'étanchéité (110) est disposé entre le cache (74) et la coque supérieure (70) ainsi que la coque inférieure (72), en particulier, le joint d'étanchéité (110) étant disposé entre le cache (74) et une rainure (102) d'un cadre extérieur (78), le cadre extérieur (78) reliant la coque supérieure (70) à la coque inférieure (72).

15. Support thermorégulateur (10) selon l'une des revendications 12 à 14,
**caractérisé en ce que**
le dispositif (32) est disposé au niveau de la jonction entre la coque supérieure (70) et la coque inférieure (72), de préférence sur le cadre extérieur (78).
